(19) Europäisches Patentamt
European Patent Office
Office européen des brevets

(11) EP 2 461 676 B1

(12) EUROPEAN PATENT SPECIFICATION

(45) Date of publication and mention of the grant of the patent:
**03.10.2012 Bulletin 2012/40**

(21) Application number: **10732361.0**

(22) Date of filing: **13.07.2010**

(51) Int Cl.:
***A01N 25/12*** *(2006.01)* ***A01N 59/16*** *(2006.01)*
***A01N 25/34*** *(2006.01)* ***C02F 1/50*** *(2006.01)*
***C02F 1/68*** *(2006.01)* ***A01N 25/10*** *(2006.01)*
***A01P 1/00*** *(2006.01)*

(86) International application number:
**PCT/EP2010/060056**

(87) International publication number:
**WO 2011/015429 (10.02.2011 Gazette 2011/06)**

(54) **Antimicrobial material for water sterilization comprising a polyamide carrier and elemental silver nanoparticles**

ANTIMIKROBIELLER STOFF ZUR WASSERSTERILISIERUNG MIT EINEM POLYAMIDTRÄGER UND ELEMTAREN SILBERPARTIKELN

Matériau antimicrobien pour la stérilisation de l’eau comprenant une charge à polyamide et des nanoparticules d’argent elementaire

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **03.08.2009 EP 09167095**

(43) Date of publication of application:
**13.06.2012 Bulletin 2012/24**

(73) Proprietor: **DSM IP Assets B.V.**
**6411 TE Heerlen (NL)**

(72) Inventors:
- **LOONTJENS, Jacobus**
**NL-6231 KK Meerssen (NL)**
- **VRINZEN, Alexander, Peter, Marie**
**NL-6231 GZ Meerssen (NL)**

(74) Representative: **Breitenstein, Tilman et al**
**DSM**
**DEC IP Delft**
**Alexander Fleminglaan 1**
**2613 AX Delft (NL)**

(56) References cited:
**EP-A- 1 767 495**
**WO-A-2004/063099**
**WO-A-2006/050477**
**US-A- 5 011 602**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 461 676 B1

## Description

The present invention relates to an antimicrobial material consisting of particles comprising an antimicrobial agent and a carrier, and more particularly an antimicrobial material that can be used to sterilize water by inhibiting the growth of microorganisms therein. The invention further relates to an article comprising the antimicrobial material and to a device comprising the antimicrobial material or the article. The invention also relates to a process for preparing the antimicrobial material and to the uses of: a) the antimicrobial material, b) the article and c) the device, in water treatment systems/appliances, water filters, swimming pools, whirlpools, water supply containers, water storage and for treatment of drinking water.

Nowadays, over 1 billion people on the planet don't have access to clean drinking water. Although 70% of our planet is water, only 2.5% of this water is available for drinking purposes. The rest is saltwater, not suited for drinking. Unsafe water and lack of basic sanitation cause 80% of all sickness and disease, and kill more people every year than all forms of violence, including war. Many people in the developing world especially women and children walk more than 3h/day to fetch water which is likely to make them sick. Those hours spent daily are crucial since they prevent people from going to school or working. Children are especially vulnerable to the consequences of unsafe water. Over 40.000 deaths of children occur every week due to unsafe water and a lack of basic sanitation. 90% of these deaths regard children under the age of 5. The problem is expected to grow given the anticipated rapid growth of human population in the years to come. Therefore, there is a desire for solutions that would allow large human populations across the planet, easy access to drinking water which in its turn would significantly limit disease spread and improve quality of life for these people.

Water sterilization (inhibition of propagation of microorganisms residing in the water) of otherwise unsafe to drink water is the key to provide a solution to this problem. Known water sterilization techniques are: a) the boiling of water, b) the use of chlorine and silver tablets, c) the use of iodine, d) filtration and e) combination of filtration and use of halogens. Unfortunately, none of these water sterilization methods provides for a combination of key parameters such as high antimicrobial efficiency, low cost and easy maintenance, free of health risks for humans associated to the particular antimicrobial agent (e.g. iodine is not recommended for people suffering from thyroid, pregnant women and children), scalable to both small and large scale that would allow its implementation on both small (domestic) and large (villages, towns, etc.) scale projects for water sterilization.

By antimicrobial agent is meant -also in the context of the present invention- a drug, chemical species, chemical, or other substance that either kills or slows down the growth of microbes. Among the antimicrobial agents are antibacterial drugs, antiviral agents, antifungal agents, and antiparisitic drugs. Silver has long been known as an antimicrobial agent. The antibacterial efficacy of silver depends on the nature and concentration of the active species, the type of bacteria, the surface area of the active species, the bacterial concentration, the concentration and/or surface area of species that could consume the active species and lower its activity, the mechanisms of deactivation, etc.

US 5011 602, discloses an antimicrobial material suitable for sterilizing water (inhibiting the propagation of microorganisms therein). The antimicrobial material disclosed in US 5011 602, comprises a carrier (amorphous aluminosilicate, activated clay, synthetic or natural crystalline aluminosilicate ,silica gel, alumina, sepiolite, clayey material), containing an antimicrobial agent (ions and compounds of metals such as silver, copper, zinc, mercury, lead, tin, etc.) and a porous fabric surrounding the carrier, allowing water and microorganisms to pass there through. Said fabric is employed to filter off the contaminants which may cause the formation of slime which is undesired for reasons explained in herein above.

Although, US 5011 602 provides a solution that prevents leakage of the antimicrobial material outside the fabric, though special attention is necessary in order to prepare a fabric that should have mechanical strength enough to withstand the weight of the antimicrobial material. In addition, the fabric should also have the right size of pores in order to balance out: a) free-flow of water and microorganisms in/out the antimicrobial material and b) clogging of the fabric's pores with contaminants in the water and slime over time. The fine-tuning of material's physical, chemical and mechanical properties becomes even more difficult if one considers that water contaminants are neither of the same nature, nor of the same concentration in water across the globe. More over, since the pores of the fabric will eventually be clogged up by water contaminants and slime, the antimicrobial material of US 5011 602 needs to be replaced with a new one. Furthermore, in case in which the carrier (which contains the antimicrobial agent) of US 5011 602 leaks from the fabric due to e.g. misuse/awkward use of fabric/antimicrobial material, or structural fault in the fabric, accidental tearing of the fabric, then the carrier will be dispersed into the water posing further health risks for humans who will eventually be drinking water supposedly free of microorganisms but with dispersed carrier material. It is also difficult to imagine such a solution to be cost-effective and be easily scaled-up for e.g. large water containers or reservoirs that are so vital in providing with water large amounts of human populations.

It would be advantageous to provide a solution for sterilizing water reserves in household and/or community reservoirs that would be robust, cost-effective, easy to scale-up and maintain present enhanced antimicrobial activity, incorporate a straightforward tracking of the antimicrobial efficacy and pose no health risks for humans.

The object of the present invention is to solve some or all of the problems or disadvantages such as identified herein with the US 5011 602.

Therefore, broadly in accordance with the invention there is provided: an antimicrobial material consisting of particles comprising an antimicrobial agent (A) and a carrier (B), wherein:

a. the antimicrobial material is a granular material;
b. at least 90% w/w of the particles have a particle size in the range of from 1.0 mm up to and including 8.0 mm measured according to the method DIN 66165-1/-2;
c. at least 90% w/w of the particles have an aspect ratio R, equal to or lower than 8;
d. the antimicrobial agent (A) comprises elemental silver nanoparticles which elemental silver nanoparticles are mixed in the carrier (B);
e. the antimicrobial agent (A) is present in an amount in the range of from 0.001% w/w up to and including 2% w/w on antimicrobial material;
f. the carrier (B) comprises a polyamide;
g. the carrier (B) is present in an amount in the range of from 98% w/w up to and including 99.999% w/w on antimicrobial material.

The effect of the present invention is that it provides an antimicrobial material which exhibits at least one, more preferably at least two, even more preferably at least three, most preferably at least four, or five of the following advantages:

i) is robust;
ii) is easy to clean from undesired deposits;
iii) has enhanced antimicrobial activity;
iv) is easy to scale-up and maintain;
v) is straightforward to track its antimicrobial activity over time;
vi) poses no health risks.

With "robust" is meant that the antimicrobial material maintains structural integrity during its use.

With "easy to clean from undesired deposits" is meant that the antimicrobial material presents, with the help of gentle shaking e.g. 10 rpm, easy- and/or self-cleaning properties since undesired deposits such as algae, can be either not attached at all onto its surface, or they can be easily removed once attached onto its surface.

With "enhanced antimicrobial activity" is meant that the antimicrobial material exhibits antimicrobial activity over longer time period.

With "easy to scale-up and maintain" is meant that the antimicrobial material can be used in household or large community applications without particular modifications of its manufacture and that no specialized technical personnel is necessary for its maintenance.

With "straightforward to track its antimicrobial potential over time" is meant that the antimicrobial material provides visual color fading directly related to changes of its antimicrobial activity.

With "poses no health risks" is meant that the concentration of silver being released by the antimicrobial material in the water is low while the antimicrobial material is still active. According to the World Health Organization (WHO) and the conclusion of the report on "Silver in Drinking Water" [originally published in Guidelines for drinking-water quality, 2nd ed., Vol. 2, Health criteria and other supporting information (WHO, Geneva 1996)], when silver salts are used to maintain the bacteriogical quality of drinking-water, concentration of silver up to 0.1 mg/litre could be tolerated without risk to health.

US 5011 602 is silent on an antimicrobial material and the advantageous effects thereof as according to the present invention.

In the present invention, an antimicrobial material is a material comprising an antimicrobial agent and when the antimicrobial material is in contact with a) microorganisms and b) water and/or oxygen, will render them inactive by killing them and/or inhibiting their propagation. If the antimicrobial material is not exposed to microorganisms and/or water and/or oxygen or to any of the above, it will still be referred to as an antimicrobial material in the context of this invention, though obviously its antimicrobial effect will not be exhibited. If the antimicrobial material is not exposed to microorganisms and/or water and/or oxygen or to any of the above it would still be able to exhibit antimicrobial activity and be suitable for antimicrobial applications.

A particle is defined as a small object that: a) has dimensions as described herein after and b) behaves as a whole unit in terms of its transport and properties. In the context of the present invention the terms granule, particle and grain will be used interchangeably.

A granular material is a conglomeration of discrete solid, macroscopic particles characterized by a loss of energy whenever the particles interact (the most common example would be friction when grains collide). The constituents

that compose granular material must be large enough such that they are not subject to thermal motion fluctuations. Thus, the lower size limit for grains in granular material is about 1 μm. In the context of the present invention, granular material refers to a material comprising granules with particle size in the range of from 1 and up to and including 8 mm, as measured according to the method described herein after.

Particles can be classified in different manners, e.g. in relation to their particle size and ranges thereof. Particle size ranges defining limits of classes of particles haven been given names for example in the Wentworth scale (or Udden-Wentworth) used in the U.S.A. and have been reported in "A scale of grade and class terms for clastic sediments", J. Geology 30:377-392 (1922) by C. K. Wentworth. The Wentworth scale is described in an abstract form in Table 1.

Table 1: Particle classification according to Wentworth scale (in bold the classes of particles the present invention refers to).

| Particle Size Range | Particle class (Wentworth) |
|---|---|
| > 256 mm | Boulder |
| 64-256 mm | Cobble |
| 32-64 mm | Very coarse gravel |
| 16-32 mm | Coarse gravel |
| 8-16 mm | Medium gravel |
| *4-8 mm* | Fine gravel |
| *2-4 mm* | Very fine gravel |
| *1-2 mm* | Very coarse sand |
| 0.5-1 mm | Coarse sand |
| 0.25-0.5 mm | Medium sand |
| 125-250 μm | Fine sand |
| 62.5-125 μm | Very fine sand |
| 3.9-62.5 μm | Silt |
| < 3.9 μm | Clay |
| < 1 μm | Colloid |

According to the Wentworth scale, the present invention refers to granular materials that belong to the following particle class classifications: very coarse sand and/or very fine gravel and/or fine gravel and/or mixtures thereof. Preferably the particles of this invention are not classified as boulders, cobbles, very coarse gravels, coarse gravels, medium gravels, coarse sand, medium sand, fine sand, very fine sand, silt, clays, colloids. It will be apparent that the granular material according to the present invention is not sand or alike, but are polymer based.

Granular materials are commercially important in applications as diverse as pharmaceutical industry, agriculture, and energy production. Some examples of granular materials are nuts, coal, sand, rice, coffee, corn flakes, fertilizer, and ball bearings.

In practice, real granular materials are always polydispersed, which means that the particles in an ensemble have different sizes. The notion of particle size distribution reflects this polydispersity. There is often a need of a certain average particle size for the ensemble of particles. There are several different ways of defining such average particle size.

The particle size distribution (PSD) - also known as grain size distribution - of a granular material, is a list of values or a mathematical function that defines the relative amounts of particles present, sorted according to size. The terms "particle size" and "particle size distribution" will be used interchangeably in the context of the present invention when used in relation to the granular material. However, as will be apparent, if the term "particle size" is used in relation to individual particles, this does not intend to have the meaning of a "particle size distribution" but just the size of the particle itself.

The PSD of a material can be important for its physical, chemical and mechanical properties. For example it can affect the strength and load-bearing properties of a granular material, the reactivity of solids participating in chemical reactions and it needs to be well-controlled. The way in which the particle size and/or PSD is expressed is usually defined by the method by which it is determined. There are several methods for measuring particle size and particle size distribution. Some of them are based on light, other on ultrasound, or electric field, or gravity, or centrifugation.

The method used to measure the particle size of the granular material according to the present invention is sieve analysis. The sieve analysis was carried out according to the method as described in DIN 66165-1/-2. According to it, the granular material is separated on sieves of different sizes. Thus, the PSD is defined in terms of discrete size ranges: e.g. "% of sample granular material has particle size in the range of 1.4 mm to 1.6 mm", when sieves of these sizes are used.

The particle size of the granular material of the present invention is measured by DIN 66165-1/-2. Preferably, 90% of the granular material is in the range of from 1.0 mm up to and including 8.0 mm, more preferably 95% of the granular material is in the range of from 1.0 mm up to and including 8.0 mm, even more preferably 97% of the granular material is in the range of from 1.0 mm up to and including 8.0 mm, even more preferably 98% of the granular material is in the range of from 1.0 mm up to and including 8.0 mm, even more preferably 99% of the granular material is in the range of from 1.0 mm up to and including 8.0 mm, even more preferably 100% of the granular material is in the range of from 1.0 mm up to and including 8.0 mm.

It is noted that for all upper and lower boundaries of any parameters given herein in ranges, the boundary value is included in each range for each parameter. All combinations of minimum and maximum values of the parameters described herein may be used to define the parameter ranges for various embodiments and preferences of the invention.

Unless the context clearly indicates otherwise, plural forms of the terms as used herein e.g. material, particle, granule, particle size, polyamide, nanoparticles, cation, etc. are to be construed as including the singular form and vice versa.

The particles of the granular material can come to various shapes, e.g. spheres, cones, cylinders (in the present invention the terms rod or rod-like, when used for particle shapes, will be used interchangeably with the term cylinder), rice-like, octahedrical, cubic, tabular or irregular, etc. In the context of the present invention, the shape of an object is defined by the aspect ratio R of the shape. The aspect ratio is defined as the ratio of its longest dimension (L) to its shortest dimension (S).

$$R = L/S \quad \text{(equation 1)}$$

The aspect ratio R is applied to two characteristic dimensions of a three-or a two-dimensional shape, such as the ratio of the longest to its shortest dimensions which can be measured by optical microscopy.

Preferably, the R is equal to or lower than 8, more preferably R is equal to or lower than 6, even more preferably R is equal to lower than 5 most preferably R is equal to or lower than 4. Implicitly, R is equal to or greater than 1. Preferably R is in the range of from 1 up to and including 5, more preferably R is in the range of from 1 up to and including 4, even more preferably R is in the range of from 1 up to and including 3, most preferably R is in the range of from 1 up to and including 2.

In one embodiment the granules of the antimicrobial material are of spherical and/or cylindrical (alternative term for cylindrical is rod-like) and/or rice-like shape and/or ellipsoidal.

In another embodiment of the present invention, at least 90% w/w of the particles have an underwater angle of friction (angle of friction measured underwater) of equal to or lower than 30°, measured according to the method UAF/OCA30 as described herein [see Examples, page 24, line 32 "Method UAF/OCA30: Method for measuring the underwater angle of friction (UAF)].

The underwater angle of friction (UAF) of a particle of the present invention is the arithmetic mean of n measurements of the angle from horizontal on which the particle will begin to slide when placed onto a round polystyrene surface with a diameter of 5.0 cm, both particle and surface being submerged in water and both being subject to a water pressure equal to the pressure generated by a cylindrical column of 25 ml of water with a diameter of 5.0 cm. The UAF is measured underwater. For measuring the UAF a video-based semi-automatic contact angle meter, the OCA 30 by DataPhysics Instruments GmbH was used. The above apparatus is typically used for the semi-automatic measurement of the wetting behavior of solids, as well as for series tests and systematic analysis. In the context of the present invention this device was used to accurately measure the underwater angle of friction of the granules of the granular material of the present invention provided in a variety of shapes and particle sizes.

A polystyrene cylindrical container, with a diameter of 5.0 cm and a height of at least 3.0 cm, can be charged with 25 ml (milliliters) water. A sample particle can be immersed into the water and left to sink at the periphery of the bottom of the container, in such a way that at the same time it would touch the wall of the container. The container can be then fixed properly on the measuring stage of the OCA30. The measuring stage is software controlled motorized adjustable in Y- and Z- axis and adjustable for accurate sample positioning. Subsequently, the measuring stage is set to start tilting. The tilting speed of the measuring stage was 2.7 °/sec. The tilting of the measuring stage is tracked by an in-line video camera. The angle at which the sample particle starts sliding from its initial position towards the non-tilted side of the measuring stage can be registered by the in-line video camera recording. The measurement is repeated

*n-1* more times (n represents the total number of measurements per granular material) every time with randomly selected particles from the granular material. The recorded -as described above- angles are averaged (summing the values of the angles and diving by *n)* and the mean (average) value of these n measurements is reported as the underwater angle of friction for the individual particles. The thus measured angle of friction of the particles of the granular material simulates better the aqueous environment under which these particles will exhibit the array of their unique properties. In real life conditions, the water pressure the granular material is being subject to can be greater than that used in measuring the underwater aqueous angle of friction. Nevertheless, given the robustness of the particles, water pressure higher than the pressure generated by a cylindrical column of 25 ml of water with a diameter of 5.0 cm would enhance their easy- and/or self-cleaning properties.

The underwater angle of friction is a measure of the amount of external force under the application of which the particle of the antimicrobial material of the present invention will start to move. A low(er) underwater angle of friction for a particle of a certain shape and form, means that the particle will start to move when subject to relative small(er) external force. A high(er) underwater angle of friction for a particle of a certain shape and form, means that the particle will start to move when subject to relative great(er) external force.

Preferably the underwater angle of friction is equal to or smaller than 30°, more preferably the underwater angle of friction is equal to or smaller than 29°, even more preferably the underwater angle of friction is equal to or smaller than 28°, even more preferably the underwater angle of friction is equal to or smaller than 27°, most preferably the underwater angle of friction is equal to or smaller than 26°. Generally, the underwater angle of friction is equal to or greater than 1°, suitably the underwater angle of friction is equal to or greater to 2°, or equal to or greater to 3°, while it is still OK when the underwater angle of friction is equal to or greater than 4°, or equal to or greater than 5°.

In another embodiment the present invention provides for an antimicrobial material wherein at least 90% w/w of the particles have an underwater angle of friction (UAF) equal to or lower than 30°. Preferably, at least 95% w/w of the particles have an underwater angle of friction (UAF) equal to or lower than 30°, more preferably at least 98% w/w of the particles have an underwater angle of friction (UAF) equal to or lower than 30°, most preferably 100% w/w of the particles have an underwater angle of friction (UAF) equal to or lower than 30°. Herein, the UAF was measured according to the method UAF/OCA30 described herein [see Examples, page 24, line 32 "Method UAF/OCA30: Method for measuring the underwater angle of friction (UAF)].

In the present invention, a granular antimicrobial material refers to a material which consists of granules and comprises an antimicrobial agent. In the context of the present invention, the terms "granular antimicrobial material" and "antimicrobial material which is granular" will be used interchangeably.

Easy- and/or self-cleaning of the particles can for example be achieved by shaking of the article/device that contains the antimicrobial material and/or by other means of shaking or vibration that would allow the particles to move against each other. Although, the applicants do not bind to a particular theory, it is believed that the movement of the particles results into scraping from each other's surface through mechanical friction any deposits that may otherwise restrict antimicrobial potential of the antimicrobial material. The lower the underwater angle of friction of a particle, the easier the particle or an amount of same particles will start moving, thus allowing enhanced friction between them and subsequent cleaning of undesired deposits on their surfaces with minimum external force applied. In this way particles of the present invention can exhibit easy- and/or self-cleaning properties, allowing for enhanced antimicrobial activity.

The antimicrobial agent (A) is present in an amount in the range of from 0.001 % w/w up to and including 2% w/w on antimicrobial material. More preferably the antimicrobial agent is present in an amount in the range of from 0.01% w/w up to and including 1.5% w/w on antimicrobial material, even more preferably the antimicrobial agent is present in an amount in the range of from 0.1% w/w up to and including 1.0% w/w on antimicrobial material.

In the present invention the antimicrobial agent comprises elemental silver (Ag°) nanoparticles. In the context of the present invention, the term elemental silver nanoparticles refers to particles which comprise elemental silver (Ag°) and suitably have an average particle size in the range of from 1 nm (nanometer) up to and including 100 nm.

The elemental silver nanoparticles of the invention preferably comprise predominantly silver (Agº), meaning that they are greater than 90% w/w silver, preferably greater than 95% w/w silver, more preferably greater than 98% silver, even more preferably greater than 99% w/w silver, most preferably greater than 99.4% w/w silver, even most preferably are 100% w/w silver. Preferably, the elemental silver nanoparticles do not have elemental Al, or Fe or Si or their corresponding ions or combinations thereof. The elemental silver nanoparticles are preferably made by pyrolyzing a chemical compound comprising silver.

In the context of the present invention by the term chemical compound comprising silver is meant any chemical compound that comprises silver as a cation, e.g. $Ag^+$, $Ag^{+2}$, etc. The compound itself may have more than species of cations, though at least one must be a silver cation. Just to name a few general types of chemical compounds that are characterized in the context of the present invention as chemical compounds comprising silver, are silver oxides, silver hydroxides, silver salts, silver complexes, silver inclusion compounds, etc. Preferably the chemical compound comprising silver are silver salts such as for example silver nitrate, silver chloride, silver acetate, silver trifluoroacetate, or mixtures of silver salts. More preferably, the chemical compound comprising silver is selected from the group of compounds

consisting of silver nitrate, silver acetate, silver trifluoroacetate and mixtures thereof.

The elemental silver nanoparticles may be in any shape including for example spherical, octahedral, conical, cubic, tabular or irregular, etc. Preferably, the elemental silver nanoparticles are not adsorbed onto and/or binded to another inorganic molecular particle or active carbon and/or ions of other metal/inorganic ions and are not exchanged for another metal/inorganic ions, the latter forming part of an inorganic molecular particle or active carbon.

The average particle size of the elemental silver nanoparticles can be measured by transmission electron microscopy (TEM). The average particle size of elemental silver nanoparticles (APSN) is the number average particle size defined as:

$$APSN= (\Sigma_i N_i D_i)/ (\Sigma_i Ni) \qquad \text{(equation 2)}$$

where $N_i$ is the number of all elemental silver nanoparticles present in three microtome samples (cuts) with dimensions of 1 mm (length) x 1 mm (width) and layer thickness in the range between 70 and 100 nm, with diameter $D_i$. The three microtome cuts are done in three randomly selected particles of the granular material.

In a preferred embodiment of the present invention, the elemental silver nanoparticles have an average particle size equal to or lower than 100 nm. Preferably the average particle size of the elemental silver nanoparticles is equal to or greater than 1 nm, more preferably is equal to or greater than 5 nm, most preferably is equal to or greater than 10 nm. Preferably the average particle size of the elemental silver nanoparticles is equal to or lower than 100 nm, more preferably is equal to or lower than 95 nm, most preferably is equal to or lower than 90 nm.

The elemental silver nanoparticles are mixed in the carrier (B). The terms "elemental silver nanoparticles" and "nanoparticles" will be used interchangeably in the context of the present invention. The elemental silver nanoparticles of the present invention when mixed in the carrier (B) can be characterized as being either dispersed or associated. In the present invention, elemental silver nanoparticles which are not touching or are not connected to any other elemental silver nanoparticle are referred to as dispersed of non-associated nanoparticles. In the present invention, elemental silver nanoparticles which are touching or are connected to at least one other elemental silver nanoparticle are referred to as "associated nanoparticles".

The % degree of dispersion (% DOD) and the degree of association (% DOA) of the elemental silver nanoparticles can be measured by transmission electron microscopy (TEM) according to sample's requirements as described herein above for the measurement of the APSN.

The relationship between the % DOD and the % DOA of the elemental silver nanoparticles into the carrier (B) is as follows:

$$\%\ DOD= 100 - \%\ DOA \qquad \text{(equation 3)}$$

The % DOA of the elemental silver nanoparticles can be measured by transmission electron microscopy (TEM) according to sample's requirements as described herein above for the measurement of the APSN. The % DOA is measured as follows:

$$\%\ DOA= 100x\ (N_{connected} / N_i) \qquad \text{(equation 4)}$$

where $N_i$ was the number of elemental silver nanoparticles present in three microtome samples (cuts) with dimensions of 1 mm (length) x 1 mm (width) and layer thickness in the range between 70 and 100 nm, and $N_{connected}$ represents the total number of distinct elemental silver nanoparticles which were connected to at least one other elemental silver nanoparticle. The three microtome cuts are done in three randomly selected particles of the granular material.

The APSN and the % DOA can be measured from the same three microtome samples or from different ones. Preferable, the APSN and the % DOA are measured from the same three microtome samples.

In a preferred embodiment of the present invention, the elemental silver nanoparticles have a % degree of association lower than 50%, more preferably lower than 40%, even more preferably lower than 30%, most preferably lower than 28%, for example lower than 25%.

In a preferred embodiment of the present invention, the elemental silver nanoparticles have a % degree of dispersion higher than 50%, more preferably higher than 60%, even more preferably higher than 70%, most preferably higher than 72%, for example higher than 75%.

In yet another embodiment of the present invention is provided an antimicrobial material wherein the elemental silver nanoparticles have an average particle size equal to or lower than 100 nm and a % degree of association lower

than 50%.

By carrier (B) is meant a polymer composition in which the antimicrobial agent (elemental silver nanoparticles) is dispersed in. Preferably, the carrier (B) does not carry the elemental silver nanoparticles of the present invention through adsorption, binding or ion exchange.

In the context of the present invention, the carrier (B) can comprise more than one polymer, i.e. a polyamide and one or more other polymers. Preferably, the carrier (B) comprises only a polyamide and no other polymer. Preferably, the carrier (B) does not comprise any crosslinker e.g. epoxies, beta-hydroxyalkylamides, triglycidyl isocyanurate (TGIC), blocked isocyanates, amino-formaldehyde resins (melamines), etc.

In the context of the present invention, a polymer is a large molecule (macromolecule) composed of repeating structural units typically connected by covalent chemical bonds. While the term polymer may refer to a large class of natural and synthetic materials with a variety of properties, in the context of the present invention the term polymer is used to describe only synthetic materials and not naturally occurring.

According to the invention, the carrier (B) comprises a polyamide. In principle a polyamide is a polymer containing monomeric units joined by (O=)C-NH-(amide) bonds. The amide bond can be produced from the condensation reaction of an amino group and a carboxylic acid or acid chloride group. A small molecule, usually water, or hydrogen chloride, is eliminated during the synthesis of polyamides. The polymer can be constituted from monomers comprising anamino group and a carboxylic acid group (the latter is called an amino acid), or the polymer can be constituted of two different bifunctional monomers, one with two amino groups, the other with two carboxylic acid or acid chloride groups. Polyamides can also be synthesized from cyclic amides (lactams) as starting monomers. In the case a lactam is used as a starting monomer no water or hydrogen chloride is eliminated during the synthesis of polyamides. Although polyamides can occur both naturally, examples being proteins, such as wool and silk, and can be made artificially through step-growth polymerization, examples being nylons, aramids, etc., in the present invention the term polyamides refers only to artificially (synthetically) prepared polyamides and not to naturally occurred ones. In the context of the present invention the term polyamide will be used interchangeably with the term nylon.

The polyamides for use as carrier in the antimicrobial material according to the invention, may comprise the polycondensation reaction product or residue of: a) at least one diamine component and at least one dicarboxylic acid component or b) at least one lactam. It will be clear to the person skilled in the art that instead of/or in combination with the dicarboxylic acid also its anhydride can be used. Hereafter, with dicarboxylic acid is also meant its anhydride.

The polyamide used in the invention can suitably be prepared from one type of diamine and one type of dicarboxylic acid or the polyamide can be prepared from for example one type of diamine combined with more than one type of dicarboxylic acid or it can be prepared from one type of dicarboxylic acid with more than one type of diamine or it can be prepared from the combination of more than one type of diamine with more than one type of dicarboxylic acid. It is however preferred to use a polyamide prepared from one to two diamines and one to two dicarboxylic acids, more preferably to use a polyamide prepared from one diamine and one dicarboxylic acid. The polyamide can also be prepared from a lactam or a mixture of lactams. Preferably the polyamide is prepared from one lactam. The polyamide may also be prepared from an amino acid or a mixture of amino acids. Preferably the polyamide is prepared from one amino acid.

Suitable dicarboxylic acids include for example dicarboxylic acids having from 3 to about 40 carbon atoms, and more preferably dicarboxylic acids selected from aromatic dicarboxylic acids preferably having 8 to 14 carbon atoms, aliphatic dicarboxylic acids preferably having 4 to 12 carbon atoms, and/or cycloaliphatic dicarboxylic acids preferably having 8 to 12 carbon atoms. The dicarboxylic acids may be branched, nonlinear or linear. Preferably, the dicarboxylic acids are linear. Examples of suitable dicarboxylic acids are for example phthalic acid, isophthalic acid, terephthalic acid, 1,4-cyclohexanedicarboxylic acid, naphthalene-2,6-dicarboxylic acid, cyclohexanediacetic acid, diphenyl-4,4'-dicarboxylic acid, phenylenedi (oxyacetic acid), sebacic acid, succinic acid, adipic acid, glutaric acid and/or azelaic acid. Preferable dicarboxylic acids are azaleic acid, adipic acid, terephthalic acid, isophthalic acid and dodecanedioic acid.

The diamine can for example be of an aliphatic, cycloaliphatic, aliphatic-aromatic or aromatic nature. The diamine component may comprise an aliphatic diamine having for example 2 to 50 carbon atoms, more preferably 2 to 30 carbon atoms , most preferably 2 to 20 carbon atoms, for example 2 to 12 carbon atoms. The aliphatic diamines may also contain aromatic groups in the molecule. With aromatic amines the glass transition temperature of the polyamide can be very high. It is therefore preferred to use aliphatic and aromatic-aliphatic amines. With aliphatic amine is meant a compound in which the amine-group is directly coupled to an aliphatic chain. With aromatic amine is meant a compound in which the amine group is directly coupled to an aromatic ring structure. The aliphatic diamines also include cycloaliphatic diamines such as for example piperazine. Examples of suitable aliphatic diamines include for example isophorondiamine, 1,2-ethylenediamine, 1,3-propylenediamine, 1,6-hexamethylenediamine, 1,12-dodecylenediamine, 1,4 cyclohexanebismethylamine, piperazine, p-xylylenediamine and/or m-xylylenediamine. The amine component may also comprise branching components to obtain branched polyamides. Suitable examples include for example di-alkylene-triamines such as for example di-ethylene-triamine, di-alkylene-tetramines, di-alkylene- pentamines, di-hexamethylene-triamine, poly- functional acids such as for example 1,3,5-benzene tricarboxylic acid, trimellitic anhydride and pyromelitic anhydride and poly- functional amino acids such as for example aspartic acid and glutamic acid. Preferable diamines are diaminobutane, m-xylylenen-diamine, trimethylhexa-methylene-diamine, hexamethylene diamine, hexanedimethylenediamine, dimethyl diaminodicyclohexylmethane.

A lactam (the noun derives from the words *lactone* + *amide)* is a cyclic amide. Prefixes indicate how many carbon atoms (apart from the carbonyl moiety) are present in the ring: β-lactam (2 carbon atoms outside the carbonyl, 4 ring atoms in total), γ-lactam (3 and 5), δ-lactam (4 and 6). Preferable lactams are caprolactam and laurinlactam.

An amino acid is a molecule containing both amine and carboxyl functional groups. In the context of the present invention, these molecules read on the following general Formula (I):

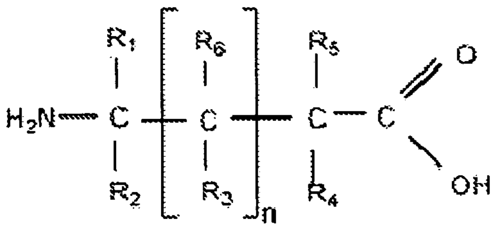

herein:

n is an integer in the range of from 0 up to and including 50, preferably n is an integer in the range of from 0 up to and including 40, more preferably n is an integer in the range of from 0 up to and including 30, even more preferably n is an integer in the range of from 0 up to and including 28, most preferably n is an integer in the range of from 0 up to and including 20, for example n is an integer in the range of from 0 up to and including 18; and $R_1$, $R_2$, $R_3$, $R_4$, $R_5$ and $R_6$ can individually be hydrogen and/or organic substituent comprising 1-50 carbon atoms, more preferably 1-30, even more preferably 1-20, most preferably 1-10, for example 1-6.

Preferably, the amino acid used for preparing the polyamide of the present invention is 11-aminoundecanoic acid.

The polyamide according to the invention can be amorphous, or semi-crystalline. In the context of the present invention, by semi-crystalline polyamide is meant that the semi-crystalline polyamide has at least one defined melting peak and melting temperature ($T_m$) (°C) measured according to ISO 11357-1/-3 (heating and cooling rate equal to 10°C/min). Suitably, the melting temperature is in the range of from 150°C up to and including 370°C, more preferably in the range of from 160°C up to and including 350°C, even more preferably in the range of from 165°C up to and including 330°C. The semi-crystalline polyamide can also have at least one glass transition temperature ($T_g$). The glass transition temperature ($T_g$) (measured according to ISO 11357-2), is preferably equal to or higher to 20°C, even more preferably is equal to or higher to 30°C, most preferably is equal to or higher to 35°C. Preferably the glass transition temperature ($T_g$) is equal to or lower than 250°C, more preferably is equal to or lower than 240°C, even more preferably is equal to or lower than 230°C, most preferably is equal to or lower than 220°C. In the context of the present invention, by amorphous polyamide is meant that the polyamide has only glass transition temperature(s) ($T_g$). Preferably the polyamide is semi-crystalline.

In the case of semi-crystalline polyamides, above their melting temperatures ($T_m$), thermoplastics like nylon are amorphous solids or viscous fluids in which the chains approximate random coils. Below $T_m$, amorphous regions alternate with regions which are lamellar crystals. The amorphous regions contribute elasticity and the crystalline regions contribute strength and rigidity. The amount of crystallinity depends on the details of formation, as well as on the kind of nylon. When nylons are extruded into fibers through pores in an industrial spinneret, the individual polymer chains tend to align because of viscous flow. If subjected to cold drawing afterwards, the fibers align further, increasing their crystallinity, and the material acquires additional tensile strength. In practice, nylon fibers are most often drawn using heated rolls at high speeds. Block nylon tends to be less crystalline, except near the surfaces due to shearing stresses during formation.

The fact that the antimicrobial agent is dispersed in carrier (B) which comprises polyamide -the latter can be easily dyed- and at the same time the carrier (B) is exposed to direct contact with water, makes easy to track the antimicrobial activity. Nylon particles are typically colorless or white depending on the type of nylon. The dispersion of the elemental silver nanoparticles into a polyamide turns yellow the particles of the granular material of the present invention. When the granular material of the present invention is immersed and left in non-sterilized water, it will exhibit its antimicrobial properties. Over time the antimicrobial activity of the granular antimicrobial material will be decreasing since the average particle size of the elemental silver nanoparticles will be decreasing due to the gradual release of $Ag^+$ cations as explained herein. As a consequence the yellow color of the granules of the antimicrobial material will be fading out over time till it is completely faded out, resulting to either colorless or white granules depending on the type of nylon

used as carrier (B). The gradual transition from yellow to either white or no color via different tones of yellow, can be visually tracked. This is a very useful and distinguishing feature of the present invention because it allows for timely maintenance e.g. replacement of the antimicrobial material without the use of expensive and sophisticated equipment, or specialized personnel, thus ensuring proper antimicrobial protection for the end-user. Even in case in which the antimicrobial material of the invention is surrounded by a container (e.g. antimicrobial material being part of a water filter), the latter may be manufactured in such a way that the color fading can still be visually tracked through perforations of particle size, the latter being such as the container to still contain the granules within it.

The polyamide may have for example a linear or nearly, branched, highly branched, star or dendritic structure. Preferably the polyamide is linear or nearly linear or branched, even more preferably the polyamide is linear or nearly linear.

Preferably, the polyamide (amorphous or semi-crystalline, neat as without impact modifiers, flame retardants, fillers, impact modifiers, plasticizers, reinforcement agents such as glass fibers, inorganic fibers, nanofillers, etc.) of the present invention has at least four, preferably at least five, more preferably has at least six, most preferably has at least seven, for example eight, out of the following seven properties:

1. Density (Kgr/$m^3$: kilogram/cubic meter) (measured according to ISO 1183) is in the range of from 1010 up to and including 3000, more preferably in the range of from 1015 up to and including 2000, even more preferably in the range of from 1020 up to and including 1800, even more preferably in the range of from 1020 up to and including 1600, most preferably in the range of from 1020 up to and including 1500, for example in the range of from 1020 up to and including 1400.
2. Elongation at break (or nominal strain at break) (%) (measured according to ISO 527-1/-2), is at least equal to/ higher to 5 %, more preferably is at least equal to or higher than 10 %, even more preferably is at least equal to or higher than 12 %, even more preferably is at least equal to or higher than 15 %. The elongation at break is equal to or lower than 500 %, more preferably is equal to or lower than 400 %, even more preferably is equal to or lower than 300 %, most preferably is equal to or lower than 300 %.
3. Tensile yield stress (MPa) (measured according to ISO 527-1/-2, to convert MPa to psi multiply by 145) is at least equal to or higher than 15 MPa, more preferably is at least equal to or higher than 20 MPa, even more preferably is at least equal to or higher than 25 MPa, even more preferably is at least equal to or higher than 30 MPa. The tensile yield strength is equal to or lower than 200 MPa, more preferably is equal to or lower than 150 MPa, even more preferably is equal to or lower than 130 MPa, most preferably is equal to or lower than 120 MPa.
4. Flexural modulus (MPa) (measured according to ISO 178) is at least equal to or higher than 900 MPa, more preferably is at least equal to or higher than 950MPa, even more preferably is at least equal to or higher than 1000 MPa, even more preferably is at least equal to or higher than 1050 MPa. The flexural modulus is equal to or lower than 6000 MPa, more preferably is equal to or lower than 5800 MPa, even more preferably is equal to or lower than 5700 MPa, most preferably is equal to or lower than 5500 MPa.
5. Flexural strength (MPa) (measured according to ISO 178) is at least equal to or higher than 10 MPa, more preferably is at least equal to or higher than 20MPa, even more preferably is at least equal to or higher than 30 MPa, even more preferably is at least equal to or higher than 40 MPa. The flexural strength is equal to or lower than 2000 MPa, more preferably is equal to or lower than 1900 MPa, even more preferably is equal to or lower than 1800 MPa, most preferably is equal to or lower than 1700 MPa.
6. Charpy notched impact strength at 23 °C (KJ/$m^2$: Kilojoule/square meter) (measured according to ISO 179/1 eA) is at least equal to or higher than 2 KJ/$m^2$, more preferably is at least equal to or higher than 3 KJ/$m^2$, even more preferably is at least equal to or higher than 4 KJ/$m^2$, even more preferably is at least equal to or higher than 5 KJ/$m^2$. The charpy notched impact strength is equal to or lower than 250 KJ/$m^2$, more preferably is equal to or lower than 200 KJ/$m^2$, even more preferably is equal to or lower than 190 KJ/$m^2$, most preferably is equal to or lower than 180 KJ/$m^2$.
7. Deflection temperature (°C) at 0.45MPa (measured according to ISO 75-1/-2), is in the range of from 130°C up to and including 300°C, more preferably in the range of from 135°C up to and including 290°C, even more preferably in the range of from 140°C up to and including 280°C, most preferably in the range of from 145° up to and including 260°C.
8. Number average molecular weight ($M_n$) (measured according to ISO 16014-3 with 1,1,1,3,3,3-hexafluoroisopropanol as eluent) is at least equal to or higher than 8000 grams/mol, more preferably is at least equal to or higher than 10000 grams/mol, even more preferably is at least equal to or higher than 12000 grams/mol, even more preferably is at least equal to or higher than 15000 grams/mol. Preferably the $M_n$ is at least equal to or lower than 100000 grams/mol, even more preferably is at least equal to or lower than 80000 grams/mol, even more preferably is at least equal to or lower than 60000 grams/mol, even more preferably is at least equal to or higher than 50000 grams/mol.

Preferably the antimicrobial material has elongation at break of at least 5%, tensile yield stress of at least 15 MPa, flexural modulus of at least 900 MPa, flexural strength of at least 10 MPa and charpy notched impact strength of at least 2 $KJ/m^2$.

The molecular weight and the viscosity associated to it, of the polyamide can play a role as per the desired end-shape and particle size of the particles, especially when extrusion is used as melt processing technique. For example, medium to high viscosity grades are used for rods of diameters greater than 3 mm, slow solidification is essential to avoid voids and cracks caused by non-uniform shrinkage. In case more complex structures are required then the extrusion of the polyamide involves extruding the polyamide into a series of interconnecting open-ended molds.

The polyamide of the present invention is preferably a thermoplastic polyamide. Thermoplastic polyamides or their compositions thereof, do not require curing [chemical reaction that proceeds via molecular weight increase, eventually polymer chains become linked together (crosslinked) into a network of infinite molecular (network formation)] when producing or fabricating parts and the only heating necessary is that required to complete melting or fusion of the polyamide. Thermoplastic polyamides or their compositions thereof do not comprise a crosslinker such as for example epoxies, beta-hydroxyalkylamides, triglycidyl isocyanurate (TGIC), blocked isocyanates, amino-formaldehyde resins (melamines), etc. because they do not require curing. When thermoplastic polyamides are subject to heat do not undergo irreversible transformation from a viscous liquid to a solid or an elastic gel or rubber. Thermoplastic polyamides have distinctively higher number average molecular weight ($M_n$) over -commonly known as-thermosetting polyamides.

The carrier (B) can comprise more than one polymers. The carrier (B) can comprise a blend of polyamides, and or a blend of the polyamide or polyamides with other polymers. The use of a blend of polymers can be in order to obtain a balance of the properties of the two materials or to reduce moisture uptake. Blends of nylon-6,6 with poly (phenylene oxide) have been most successful, but blends of nylon-6,6 and nylon-6 with polyethylene or polypropylene may also be used. Preferably, the carrier (B) comprises polyamide blends prepared from polyamides of the present invention.

According to the present invention, the carrier (B) can also comprise additives. Additives that can form part of the carrier (B), include additives commonly known to the skilled person such as lubricants, nucleants, stabilizers, impact modifiers, flame retardants, impact modifiers, fillers, plasticizers, reinforcement agents such as glass fibers, inorganic fibers, nanofillers, etc. The latter (nanofillers) are materials in which nanometer size silicate layers are uniformly dispersed into polyamides to give high levels of reinforcement at very low levels (e.g. 5%). The layer-type clay minerals are treated to make the layers separate allowing them to be incorporated into the polymer structure (intercalation). Initial developments involved incorporation during nylon polymerization but extrusion compounding routes have now been developed. Some advantages of this technology are low density and isotropic shrinkage. The additives can also be of polymeric nature.

Preferably, the carrier (B) comprises only polymers and additives, more preferably the carrier (B) comprises only one polymer and additives, even more preferably the carrier (B) comprises a polyamide and additives, for example the carrier (B) comprises only one polyamide, no other polymer and additives.

In a yet another preferred embodiment the particle size of the granular material is in the range of from 1.5 mm up to and including 5.0 mm, the aspect ratio R is in the range of from 1.0 up to and including 6.0, the particles have an underwater angle of friction in the range of from 2 up to and including 28°, the amount of the antimicrobial agent (A) is in the range of from 0.001% w/w up to and including 1.8% w/w on antimicrobial material, and the amount of the carrier (B) is in the range of from 98.2% w/w up to and including 99.999% w/w on antimicrobial material.

In yet another preferred embodiment, the carrier (B) is a polyamide selected from the group of nylon-6, nylon-11, nylon-12, nylon-6,6, nylon-4,6, nylon-6,9, nylon-6,12, nylon-66, nylon-6T, nylon-6-6-T, nylon-4-6-T, nylon-MXD, nylon-6T,61, nylon-MXD-6, nylon-PDA-T, nylon-6-3-T (polyamide NDT/INDT), nylon-61, nylon-6-G, nylon-12-G, a block copolymer of nylon-12 and poly-THF (PEBAX), polyphthalamide, or mixtures thereof. Preferably, the polyamide is selected from the group consisting of nylon-6, nylon-4,6 or nylon-6,6 and mixtures thereof. Typical thermoplastic polyamides are for example those commercialized under the following trade names: Akulon, Stanyl, Zytel, Celanese, Vydyne, Capron, Ultramid, Grillon, Nycoa, Rilsan, Amodel, Orgalloy, Technyl, Durethan, Vestamid, Trogamid, Technyl, Radilon, Leona, Novamid, Reny, Arlen, Amilan, Ube Nylon, Unitika, Grillamid, Grivory, Beetle, Polynil, etc. Preferably the polyamide of the present invention is Zytel, or Akulon or Stanyl or mixtures thereof, more preferably Akulon or Stanyl or mixtures thereof are to be used as carriers, even more preferably Akulon or Stanyl is to be used as carrier (B).

The present invention also provides for a method for the preparation of a granular antimicrobial material according to the present invention, comprising the following steps:

a. dry-mixing a polyamide with a chemical compound comprising silver as cation, thereby forming a premix;
b. blending and heating the premix for a time and at a temperature which are:

i) suitable to flow out the polyamide without decomposing it;
ii) suitable to decompose the chemical compound;

c. extruding, cooling and cutting;

d. collecting the granules with particle size smaller than 8 mm and greater than 1 mm.

Nylons can be extruded on conventional equipment and with settings known to the person skilled in the art. The following can for example be settings for nylon extrusion, although the invention is not limited to these settings only. The extruder drive should be capable of continuous variation over a range of screw speeds. Nylon often requires a high torque at low screw speeds; typical power requirements would be a 7.5-kW motor for a 30-mm machine or 25-kW for a 60-mm one. A nylon screw is necessary and should not be cooled. Compression ratios are for example between 3.5:1 and 4:1 for nylon-6,6 and nylon-6; between 3:1 and 3.5:1 for nylon-11 and nylon-12. The length-to-diameter ratio of the screw should be greater than 15:1; at least 20:1 is recommended for nylon-6,6, and 25:1 for nylon-12. Most extrusion operations require high viscosity (high molecular weight) nylon in order to give a high melt strength to maintain the shape of the extrudate.

The granular antimicrobial material thus produced, can be used directly for small/households uses e.g. water carafes, and/or large communal water reservoirs, providing enhanced antimicrobial activity and effective sterilization of the water.

In yet another embodiment, the present invention provides for a material obtained and/or obtainable by the method as described herein above (on page 21, lines 15 to 25).

In another embodiment, the present invention provides for an article that comprises the granular antimicrobial material of the present invention and an enveloping body containing the granular antimicrobial material. In the context of the present invention, an article is an individual object or item or element of a class designed to serve a purpose or perform a special function and can stand alone. The enveloping body may be flexible, foldable, drapable, or rigid. The enveloping body can be made of metal, wood, glass, plastic, textile, ceramic, mixtures thereof, etc. The enveloping body may have an open structure, such as an open bag, or a closed structure, such as a can closed with a lid. The enveloping body may be able to hold water thus not being permeable to water e.g. a container, or the enveloping body may allow water to flow through it, thus being permeable to water, e.g. a bag made of textile, or a perforated canister. The enveloping body suitably is made of a material having a porous structure, such as textile or perforated metal sheet. Exemplary enveloping bodies include but are not limited to containers, bags, baskets, bowls, cases, boxes, bags, baskets, disks, cases, cardboxes, etc.

In another embodiment, the present invention provides for an article as described herein, wherein the enveloping body is a container. According to the present invention the container is an enveloping body which can hold water. Exemplary containers include but are not limited to cartons, bottles, cans, jars, vials, jerry cans, capsules, non-perforated canisters, storage tanks, rainwater tanks.

In yet another embodiment, the present invention provides for an article as described herein, wherein the enveloping body is a porous water-permeable enveloping body, which porous water-permeable enveloping body has pores with a diameter such as to prohibit the granular antimicrobial material to spill out from the porous water-permeable enveloping body.

In a further embodiment, the present invention provides for a device consisting of an assembly of parts, comprising one part containing either an article according to the present invention wherein the enveloping body is a porous water-permeable enveloping body, which porous water-permeable enveloping body has pores with a diameter such as to prohibit the granular antimicrobial material to spill out from the porous water-permeable enveloping body or the granular antimicrobial material of the present invention. According to the present invention, a device is a piece of equipment or a mechanism designed to serve a special purpose or perform a special function and can consist of more than one parts (assembly of parts). For example the device can be an apparatus used for water treatment and in particular for treatment of drinking water, or an apparatus that uses treated water.

In a particular embodiment, the device according to the invention is a water purification device comprising an expandable water collecting reservoir having a variable volume, wherein at least part of the surface of the reservoir comprises a filter area, the filter area being permeable to water and allowing water to enter the water collecting reservoir when the filter area is submerged in water. The device may further comprise a mechanically driven expansion means for exerting a force for increasing the volume of the water collecting reservoir and thereby drawing water through the filter area into the water collecting reservoir. Such a device is described in WO 2009/073994, which is incorporated herein by reference. In use, the water collecting device is submerged and the expansion means is released, drawing water into the water collecting device through the filter area. The water purification device can be provided with the granular antimicrobial material according to the invention residing in the expandable water collecting reservoir. Once the water collecting device is submerged and the expansion means is released, the water is not only purified by filtration, but also further sterilized by the granular antimicrobial material, thereby making the sterilization by the granular antimicrobial material even more effective.

In yet another embodiment, the present invention provides for use of:

a) a granular antimicrobial material according to the present invention; or
b) an article according to the present invention; or
c) a device according to the present invention; or

for water treatment and in particular for treatment of drinking water. Exemplary uses include, but are not limited to water treatment systems/appliances, water filters, swimming pools, whirlpools, water supply containers, water storage tanks, rainwater tanks, dishwashers.

In a further embodiment, the present invention provides for use of a granular antimicrobial material according to the present invention, for antimicrobial applications, wherein the granular antimicrobial material is used in an amount and in a format that allows the granular antimicrobial material to exhibit its antimicrobial activity.

Yet, another aspect of the invention is the antimicrobial material according to the Examples 1 to 4, described herein.

Further aspects of the invention and preferred features thereof are given in the claims herein.

The invention will now be illustrated by the following examples without however being limited thereto.

## EXAMPLES

### Analytical Methods and Techniques

### Measurement of particle size of the granular material

The particle size of the granular material was measured on Retsch AS 200 Control sieving machine according to the method described in DIN 66165-1/-2. The parameters of the sieve analysis according to DIN 66165-1/-2 are given below:

| | |
|---|---|
| Sieving motion | three dimensional |
| Amplitude | 1.5 mm |
| Sieving interval | 10 seconds |
| Sieving type | dry |
| Sieving period | 10 minutes |

### Measurements of properties of the carrier (B)

The density of the carrier (B) was measured according to ISO 1183. The melting temperature (°C) of the carrier (B) as measured according to ISO 11357-1/-3 (heating and cooling rate equal to 10 °C/min). The glass transition temperature (°C) ($T_g$) of the carrier (B) was measured according to ISO 11357-2 (heating and cooling rate equal to 10°C/min). The elongation at break (%) of the carrier (B) was measured according to ISO 527-1. The flexural modulus (MPa) of the carrier (B) was measured according to ISO 178. The charpy notched impact strength ($KJ/m^2$) of the carrier (B) was measured according to ISO 179/1eA. The tensile yield stress (MPa) of the carrier (B) was measured according to ISO 527-1/-2 (to convert MPa to psi multiply by 145). The deflection temperature (°C) at 0.45 MPa of the carrier (B) was measured according to ISO 75-1/-2 and the number average molecular weight ($M_n$) of the carrier (B) was measured according to ISO 16014-3 with 1,1,1,3,3,3-hexafluoroisopropanol as eluent.

### Measurement of the particle size of the elemental silver nanoparticles

The particle size of the elemental silver nanoparticles and the % degree of association were measured on a transmission electron microscope (TEM) type "EFTEM LEO 912" (Leo Co.). The TEM investigations were performed on microtome cuts having layer thickness in the range between 70 and 100 nm.

The average particle size of elemental silver nanoparticles (APSN) was determined according to the following equation:

$$APSN= (\Sigma_i N_i D_i)/ (\Sigma_i Ni)$$

where $N_i$ is the number of elemental silver nanoparticles present in three microtome samples (cuts) with dimensions of 1 mm (length) x 1 mm (width) and layer thickness in the range between 70 and 100 nm, with diameter $D_i$. The three microtome cuts were done in three randomly selected particles of the granular material.

Measurement of the % degree of association of the elemental silver nanoparticles

The % degree of association (% DOA) of the elemental silver nanoparticles was measured as follows:

$$\% \text{ DOA} = 100x\ (N_{connected} / N_i)$$

where $N_i$ was the number of elemental silver nanoparticles present in three microtome samples (cuts) with dimensions of 1 mm (length) x 1 mm (width) and layer thickness in the range between 70 and 100 nm, and $N_{connected}$ represents the total number of distinct elemental silver nanoparticles which were connected to at least one other elemental silver nanoparticle. The three microtome cuts were done in three randomly selected particles of the granular material.

The APSN and the % DOA were measured from the same three microtome samples.

Method UAF/OCA30: Method for measuring the underwater angle of friction (UAF)

The underwater angle of friction (UAF) of a particle of the present invention is the arithmetic mean of n measurements of the angle from horizontal on which the particle will begin to slide when placed onto a round polystyrene surface with a diameter of 5.0 cm, both particle and surface being submerged in water and both being subject to a water pressure equal to the pressure generated by a cylindrical column of 25 ml of water with a diameter of 5.0 cm.

The UAF was measured underwater. For measuring the UAF a video-based semi-automatic contact angle meter, the OCA 30 by DataPhysics Instruments GmbH was used. The OCA 30 instrument is typically used for the semi-automatic measurement of the wetting behavior of solids, as well as for series tests and systematic analysis. In the context of the present invention this device was used to accurately measure the underwater angle of friction of the granules of the granular material of the present invention provided in a variety of shapes and particle sizes.

A polystyrene cylindrical container [5.0 cm x 2.0 cm, (diameter) x (height)] was charged with 25 ml water. A sample particle was immersed into the water and left to sink at the periphery of the bottom of the container, in such a way that it would touch the wall of the container. The container was then fixed properly on the measuring stage of the OCA30. The measuring stage is software controlled motorized adjustable in Y- and Z- axis and adjustable for accurate sample positioning. Subsequently, the measuring stage is set to start tilting. The tilting speed of the measuring stage was 2.7 °/sec. The tilting of the measuring stage is tracked by an in-line video camera. The angle at which the sample particle starts sliding from its initial position towards the non-tilted side of the measuring stage is registered by the in-line video camera recording. The measurement is repeated 4 more times (5 measurements in total per granular material) every time with randomly selected particles from the granular material. The recorded -as described above- five angles are averaged (summing the values of the angles and diving by 5) and the mean (average) value of these 5 measurements is reported as the underwater angle of friction for the particles.

Methods for assessing antimicrobial activity

i) Method for assessing antimicrobial activity after 24 hours

Antimicrobial activity tests were performed with *E-coli* ATCC strain and *Staphylococcus Aureus* ATCC strain for a time period of 24 hours.

Antimicrobial material of the present invention (5 grams) was added to 50 ml phosphate buffer (5 mM, pH=7.0), containing about $10^4$ cfu/ml bacteria, while shaking (320 rpm) at room temperature (23 °C). The shaking was continued for 24 hours at room temperature. After this time period, the solution was diluted with buffer solution (10 and 100 times) and 0.1 ml was spread over a LB-agar medium in a Petri dish, (LB-agar per liter: 10 grams Bacto Trypton; 5 grams Bacto Yeast extract; 5 grams, NaCl; 15 grams Bacto-Agar) and the cfu (colony forming units)/ml was recorded and reported. The higher the cfu/ml, the lower the antimicrobial activity of the tested antimicrobial material is.

ii) Method for assessing prolonged antimicrobial activity

Prolonged antimicrobial activity tests were performed with *E-coli* ATCC strain for a time period of 90 days.

Antimicrobial material of the present invention (5 grams) was added in 1000 ml tap water. A series of three thus prepared mixtures (granular material of the present invention and tap water) was prepared for each granular material tested. As time zero for this experiment was taken the time upon the completion of the preparation of the above mixtures. Throughout the duration of the experiment, twice a week the tap water was being refreshed.

After 30 days, the water of one of the mixtures was removed, and the granular material was added to 50 ml

phosphate buffer (5 mM, pH=7.0), containing about $10^4$ cfu/ml bacteria, while shaking (320 rpm) at room temperature (23 °C). The shaking was continued for 24 hours at room temperature. After this time period, the solution was diluted with buffer solution (10 and 100 times) and 0.1 ml was spread over a LB-agar medium in a Petri dish, (LB-agar per liter: 10 grams Bacto Trypton; 5 grams Bacto Yeast extract; 5 grams, NaCl; 15 grams Bacto-Agar) and the cfu (colony forming units)/ml was recorded and reported. The same procedure as this applied for assessing the antimicrobial activity after 30 days was repeated after 60 and 90 days with the remaining two mixtures (one used for the measurement after 60 days and the other for the measurement after 90 days).

The higher the cfu/ml, the lower the antimicrobial activity of the tested antimicrobial material is.

Properties of Akulon™ F223-D [carrier (B)]

Akulon™ F223-D (nylon-6) was used as carrier (B) in the context of the present invention. Dry Akulon™ F223-D had the following properties:

$T_m$: 220 °C;
$T_g$: 60 °C;
$M_n$: 20000 grams/mol;
Density: 1130 Kgr/$m^3$;
Elongation at break: 20 %;
Tensile yield stress: 85 MPa;
Flexural modulus: 2600 MPa;
Flexural strength: 100 MPa;
Charpy notched impact strength at 23 °C: 8 KJ/$m^2$;
Deflection temperature at 0.45 MPa: 185 °C.

Preparation of reference granular non-antimicrobial material (Ref A), comparative non-granular antimicrobial (Comp A) and new granular antimicrobial materials (Examples 1 to 4)

Ref A: Reference granular non-antimicrobial material

Akulon™ F223-D granulate was dried overnight at 80 °C. Subsequently, the dried Akulon™ F223-D granulate was dosed to a twin screw extruder, Berstorff ZE 25/48D equipped with (B).24 RM65 03 screws, a 2 mm ×3 mm die, a standard cooling bath, four collection units and a granulator (Scheer type SGS 50 E). The temperature setting was 80, 240, 250, 260, 280, 280 and 290 °C. The residence time was set for 160 seconds.

Upon extrusion, the size of the collected white rod-like granules (reference non-antimicrobial material) were approximately 2 mm x 3.5 mm (diameter x length).

The aspect ratio (R) of these granules was equal to 1.75.

Comp A: comparative non-granular antimicrobial material

Silver nitrate (500 grams) was dissolved in 500 ml water. A portion (200 ml) of the thus produced aqueous solution of silver nitrate was sprinkled on 5 kgr of Akulon™ F223-D granulate (the latter had been previously dried at 80 °C, overnight) in a tumbler. During tumbling, the aqueous solution of silver nitrate was absorbed by the Akulon™ F223-D granulate and the mixture remained free-flowing upon tumbling. Subsequently, the thus produced mixture was dosed to a twin screw extruder, Berstorff ZE 25/48D equipped with (B).24 RM65 03 screws, a 2 mm ×3 mm die, a standard cooling bath, four collection units and a granulator (Scheer type SGS 50 E). The temperature setting was 80, 240, 250, 260, 280, 280 and 290 °C. The residence time was set for 160 seconds. Upon extrusion, the size of the collected rod-like granulate particles were approximately 2 mm x 3.5 mm (diameter x length). The grains were then hot-pressed at 240 °C for 3 minutes into mould sheets, each measuring 100 mm x 100 mm x 1 mm (length x width x height).

Yellow platelets each measuring 10 mm x 20 mm x 1 mm (length x width x height) were obtained upon cutting the sheet moulds with a diamond saw blade.

The aspect ratio (R) of these platelets was equal to 20.00.

Example 1: new granular antimicrobial material

Silver nitrate (500 grams) were dissolved in 500 ml water. A portion (200 ml) of the thus produced aqueous solution of silver nitrate was sprinkled on 5 kgr of Akulon™ F223-D granulate (the latter had been previously dried at 80 °C, overnight) in an operating tumbler. During tumbling, the aqueous solution of silver nitrate was absorbed by the

Akulon™ F223-D granulate and the mixture remained free-flowing upon tumbling. Subsequently, the thus produced mixture was dosed to a twin screw extruder, Berstorff ZE 25/48D equipped with (B).24 RM65 03 screws, a 2 mm ×3 mm die, a standard cooling bath, four collection units and a granulator (Scheer type SGS 50 E). The temperature setting was 80, 240, 250, 260, 280, 280 and 290 °C. The residence time was set for 160 seconds.

Upon extrusion, the size of the collected yellow rod-like granules were approximately 2 mm x 3.5 mm (diameter x length).

The aspect ratio (R) of these granules was equal to 1.75.

The amount of silver is 1.25 % w/w of the granular material.

The particle size of the antimicrobial material of Example 1 was measured by sieve analysis according to the method described in DIN66165-1/-2. The sample size was 13.17 grams and the sieving weight loss was 0.00 grams. The results of the sieve analysis of the granular material of Example 1 are presented in Table 2.

Table 2: Sieve analysis results of the granular material of Example 1.

| SIEVE ANALYSIS RESULTS | | | | |
|---|---|---|---|---|
| Grain class (grain size) | Mass Fraction | | Cumulative | |
| (mm) | (gram) | (%) | Q3 (%) | 1-Q3 (%) |
| < 1.40 | 0.03 | 0.2 | 0.2 | 99.8 |
| 1.40-1.60 | 0.04 | 0.3 | 0.5 | 99.5 |
| 1.60-1.70 | 0.03 | 0.2 | 08 | 99.2 |
| 1.70-1.80 | 0.78 | 5.9 | 6.7 | 93.3 |
| 1.80- 2.00 | 11.86 | 90.1 | 96.7 | 3.3 |
| 2.00- 2.36 | 0.36 | 2.7 | 99.5 | 0.5 |
| 2.36- 2.50 | 0.04 | 0.3 | 99.8 | 0.2 |
| 2.50- 2.80 | 0.01 | 0.1 | 99.8 | 0.2 |
| 2.80- 3.15 | 0.02 | 0.2 | 100.0 | 0.0 |
| > 3:15 | 0.00 | 0.0 | 100.0 | 0.0 |

The x(Q=10.0 %), x(Q=50.0 %), x(Q=90.0 %) values, the $X_m$ (mean particle size) and the standard deviation or span value of the $X_m$ of the granular material of Example 1 calculated from the sieve analysis results, were as follows :

| | |
|---|---|
| x(Q=10.0 %): | 1.807 mm; |
| x(Q=50.0 %): | 1.896 mm ; |
| x(Q=90.0 %): | 1.985 mm ; |
| $x_m$ (mean particle size): | 1.898 mm, standard deviation : 0.094. |

The underwater angle of friction of:

a. the rod-like (2 mm x 3.5 mm) granules of the granular material of Example 1 and

b. the platelets (20 mm x 10 mm x 1 mm) of the non-granular material of CompA, were measured according to UAF/OCA-30 method, described herein. The results are presented in Table 3.

Table 3: Underwater angle of friction (UAF) of the particles of the non-granular material of Comp A and the granular material of Example 1.

| Material | Particle shape | Aspect Ratio | UAF (°)* | Mean UAF (°)** | Standard deviation UAF (°) |
|---|---|---|---|---|---|
| Comp A | platelet | 20.00 | 34.0 | 32.8 | 0.8 |
| Comp A | platelet | 20.00 | 33.0 | | |
| Comp A | platelet | 20.00 | 32.0 | | |
| Comp A | platelet | 20.00 | 32.0 | | |
| Comp A | platelet | 20.00 | 33.0 | | |
| Example 1 | rod-like | 1.75 | 25.0 | 24.0 | 1.2 |
| Example 1 | rod-like | 1.75 | 25.0 | | |
| Example 1 | rod-like | 1.75 | 24.0 | | |
| Example 1 | rod-like | 1.75 | 22.0 | | |
| Example 1 | rod-like | 1.75 | 24.0 | | |
| *UAF (°): underwater angle of friction of individual measurements<br>**Mean UAF (°): underwater angle of friction of the particles of the corresponding material. | | | | | |

From Table 3, it is clear that the rod-like granules of the antimicrobial material of Example 1 move much easier when immersed into a water container upon comparing them to the platelets of the antimicrobial material of Comp A.

The average particle size of the elemental silver nanoparticles (measured as described herein) was about 20 nm.

The % degree of association (% DOA) of the elemental silver nanoparticles was lower than 40 %.

Example 2: new granular antimicrobial material

Akulon™ F223-D granulate was dried at 80 °C overnight. In an operating tumbler, paraffin oil (0.25 % w/w of the Akulon™ F223-D granulate) was sprinkled over the dried Akulon™ F223-D granulate. Upon a certain time period by which the dried Akulon™ F223-D granulate was covered with paraffin oil, silver nitrate (in powder form) (2 % w/w of the Akulon™ F223-D granulate) was sprinkled over the dried Akulon™ F223-D granulate covered with paraffin oil for a time period enough to cause the silver nitrate to stick onto the surface of the dried Akulon™ F223-D granules which as explained were covered with paraffin oil. The thus produced mixture remained free-flowing upon tumbling. Subsequently, the aforementioned mixture was dosed to a twin screw extruder, Berstorff ZE 25/48D equipped with (B).24 RM65 03 screws, a 2 mm x3 mm die, a standard cooling bath, four collection units and a granulator (Scheer type SGS 50 E). The temperature setting was 80, 240, 250, 260, 280, 280 and 290 °C. The residence time was set for 160 seconds.

Upon extrusion, the size of the collected yellow rod-like granules were approximately 2 mm x 3.5 mm (diameter x length).

The aspect ratio (R) of these granules was equal to 1.75.

The amount of silver is 1.25 % w/w of the granular material.

Example 3: new granular antimicrobial material

Akulon™ F223-D granulate was cooled down with carbon dioxide ice, and grinded. The Akulon™ F223-D fluff thus produced, it was dried at 80 °C overnight. In an operating tumbler, silver nitrate (in powder form) (2 % w/w of the Akulon™ F223-D granulate) was sprinkled over the dried Akulon™ F223-D fluff. During tumbling, the silver nitrate was mixed reasonably well with the Akulon™ F223-D fluff, providing a relatively homogeneous mixture. The mixture remained free-flowing upon tumbling. Subsequently, the thus produced mixture was dosed to a twin screw extruder, Berstorff ZE 25/48D equipped with (B).24 RM65 03 screws, a 2 mm ×3 mm die, a standard cooling bath, four collection units and a granulator (Scheer type SGS 50 E). The temperature setting was 80, 240, 250, 260, 280, 280 and 290 °C. The residence time was set for 160 seconds.

Upon extrusion, the size of the collected yellow rod-like granules were approximately 2 mm x 3.5 mm (diameter x length).

The aspect ratio (R) of these granules was equal to 1.75.

The amount of silver is 1.25 % w/w of the granular material.

Example 4: new granular antimicrobial material

Akulon™ F223-D granulate was dried at 80 °C overnight. In an operating tumbler, paraffin oil (0.10 % w/w of the Akulon™ F223-D granulate) was sprinkled over the dried Akulon™ F223-D granulate. Upon a certain time period by which the dried Akulon™ F223-D granulate was covered with paraffin oil, silver nitrate (in powder form) (160 ppm w/w of Akulon™ F223-D granulate) was sprinkled over the dried Akulon™ F223-D granulate covered with paraffin oil for a time period enough to cause the silver nitrate to stick onto the surface of the dried Akulon™ F223-D granules which as explained were covered with paraffin oil. The thus produced mixture remained free-flowing upon tumbling. Subsequently, the aforementioned mixture was dosed to a twin screw extruder, Berstorff ZE 25/48D equipped with (B).24 RM65 03 screws, a 2 m ×3 mm die, a standard cooling bath, four collection units and a granulator (Scheer type SGS 50 E). The temperature setting was 80, 240, 250, 260, 280, 280 and 290 °C. The residence time was set for 160 seconds.

Upon extrusion, the size of the collected yellow rod-like granules were approximately 2 mm x 3.5 mm (diameter x length).

The aspect ratio (R) of these granules was equal to 1.75.

The amount of silver is 100 ppm (= 0.01 % w/w) of the granular material.

Example 5: Silver release over time

Granular antimicrobial material (5 grams) was suspended in 50 ml water at room temperature (23 °C), whilst gently shaken. The concentration of silver cation ($C_{Ag+}$) in the aqueous solution was measured by atomic absorption over time.

The concentration of silver cation ($C_{Ag+}$) in an aqueous solution was measured for the granular antimicrobial materials of Example 1, Example 2 and Example 3, respectively. Table 4 presents the silver release over time of the granular materials of Examples 1 to 3.

Table 4: Silver release over time ($C_{Ag+}$ vs. time) of the granular materials of Examples 1 to 3.

| Time (hours) | Granular Material | | |
|---|---|---|---|
| | Example 1 | Example 2 | Example 3 |
| | $C_{Ag+}$ (ppb)* | $C_{Ag+}$ (ppb) | $C_{Ag+}$ (ppb) |
| 1 | 10 | 12 | 12 |
| 3 | 25 | 28 | 24 |
| 5 | 30 | 33 | 27 |
| 8 | 35 | 38 | 32 |
| 16 | 38 | 42 | 33 |
| 24 | 40 | 45 | 38 |
| 48 | 42 | 45 | 40 |
| 72 | 41 | 44 | 42 |
| *ppb: parts per billion | | | |

From the data shown in Table 4, it is clear that the concentration of silver cation ($C_{Ag+}$) in the aqueous solution reaches an equilibrium since it levels off upon approximately 24h. This important finding demonstrates the unique property of the granular antimicrobial material to maintain $C_{Ag+}$lower than 0.1 mg/litre (milligram/litre) which is the highest recommended concentration according to the World Health Organization (WHO) that could be tolerated without risk to health, as explained herein. Moreover, this phenomenon is independent from the three methods of preparation of the granular antimicrobial material (Examples 1 to 3).

Examples 6 to 10: Assessment of antimicrobial activity of the granular materials of Examples Ref A and Examples 1 to 4, after 24 hours

The assessment of the antimicrobial activity of the granular materials of Examples Ref A and Examples 1 to 4 was carried out according to the method described herein (see Examples, on p. 25, line 28, "i) Method for assessing antimicrobial activity after 24 hours").The results of the antimicrobial activity of the granular antimicrobial materials of

Examples Ref A and Examples 1 to 4 are presented in Table 5 (Examples 6 to 10).

Table 5: Results of the antimicrobial activity of the granular antimicrobial materials of Examples Ref A and Examples 1 to 4.

| Blank Solution or Granular Material | | Example 6 | Example 7 | Example 8 | Example 9 | Example 10 |
|---|---|---|---|---|---|---|
| | | *E-Coli* ATCC strain | | | | *Staphylococcus Aureus* ATCC strain |
| | | cfu/ml | cfu/ml | cfu/ml | cfu/ml | cfu/ml |
| Blank Solution | Blank A[1] | 6900 | 7000 | 7200 | 8900 | ——— |
| | Blank B[2] | ——— | ——— | ——— | ——— | 7000 |
| Granular Material | Ref A | 6900 | 7000 | 7.200 | 8900 | 7000 |
| | Example 1 | 0 | ——— | ——— | ——— | ——— |
| | Example 2 | ——— | 0 | ——— | ——— | 0 |
| | Example 3 | ——— | ——— | 0 | ——— | ——— |
| | Example 4 | ——— | ——— | ——— | 0 | ——— |

[1]Blank A: a solution without any granular material but with the same amount of *E-coli* ATCC strain

[2]Blank B: a solution without any granular material but with the same amount of *Staphylococcus Aureus* ATCC strain.

From the data presented in Table 5, it becomes clear that the granular materials of the present invention do present antimicrobial activity since all *E-coli* and *Staphylococcus Aureus* bacteria were killed after been exposed for 24 hours to the granular materials of Examples 1 to 4, respectively.

Example 11: Assessment of prolonged antimicrobial activity of the granular materials of Examples Ref A and Example 1

The assessment of the prolonged antimicrobial activity of the granular materials of Examples Ref A and Example 1 was carried out according to the method described herein (see Examples, on p. 26, line 5, "ii) Method for assessing prolonged antimicrobial activity"). The results (cfu/ml vs. time) up to 3 months are shown in Table 6.

Table 6: Results of prolonged antimicrobial activity of the granular materials of Examples Ref A and Example 1.

| Time (days) | Blank Solution or Granular Material | | E-Coli ATCC stain |
|---|---|---|---|
| | | | cfu/ml |
| 30 | Blank Solution | Blank* | 9100 |
| | Granular Material | Ref A | 9100 |
| | | Example 1 | 0 |
| 60 | Blank Solution | Blank | 8900 |
| | Granular Material | Ref A | 8900 |
| | | Example 1 | 0 |

(continued)

| Time (days) | Blank Solution or Granular Material | | E-Coli ATCC stain |
|---|---|---|---|
| | | | cfu/ml |
| 90 | Blank Solution | Blank | 9050 |
| | Granular Material | Ref A | 9050 |
| | | Example 1 | 0 |
| *Blank: a solution without any antimicrobial material but with the same amount of *E-coli* ATCC strain. | | | |

From the data presented in Table 6, it becomes clear that the granular materials of the present invention provide prolonged antimicrobial activity since all *E-coli* bacteria were killed after been exposed to the granular material of Example 1.

The color of the granules of the material of Example 1 after 90 days was still yellow presenting no visually inspectable deviation from the yellow shade of freshly prepared particles.

Examples 12-13: Assessment of easy- and/or self-cleaning of the granular material of Examples Comp A and Example 1

Example 12

An Erlenmeyer flask was charged with granular material of Comp A (5 grams) and 1000 ml tap water. The Erlenmeyer flask was placed onto an automatic shaker and then they all together were placed onto a windowsill, such as the flask to be exposed to direct sunlight. During the duration of the experiment the flask was being subject to gentle and continuous shaking at 10 rpm. The flask was inspected for signs of algae after a time period of two weeks and one month. After two weeks there were signs of algae on the platelets of the antimicrobial material of Comp A. After a month algae has covered most of the surface of the platelets of the antimicrobial material of Comp A.

Example 13

An Erlenmeyer flask was charged with granular material of Example 1 (5 grams) and 1000 ml tap water. The Erlenmeyer flask was placed onto an automatic shaker and then they all together were placed onto a windowsill, such as the flask to be exposed to direct sunlight. During the duration of the experiment the flask was being subject to gentle and continuous shaking at 10 rpm. The flask was inspected for signs of algae after a time period of two weeks and one month. After two weeks there were no algae on the rod-like granules of the antimicrobial material of Example 1. Even after a month no algae has covered the surface of the granules of the antimicrobial material of Example 1.

Upon comparing the results from Examples 12 and 13, it becomes clear that the granular material of Example 1 consisting of rod-like granules with an aspect ratio R of 1.75, showed enhanced easy- and self-cleaning property over the antimicrobial material of Comp A consisting of platelets with aspect ratio R of 20.00.

**Claims**

**1.** An antimicrobial material consisting of particles comprising an antimicrobial agent (A) and a carrier (B), **characterized in that**:

a. the antimicrobial material is a granular material;
b. at least 90% w/w of the particles have a particle size in the range of from 1.0 mm up to and including 8.0 mm measured according to the method DIN 66165-1/-2;
c. at least 90% w/w of the particles have an aspect ratio R, equal to or lower than 8;
d. the antimicrobial agent (A) comprises elemental silver nanoparticles which elemental silver nanoparticles are mixed in the carrier (B);
e. the antimicrobial agent (A) is present in an amount in the range of from 0.001 % w/w up to and including 2% w/w on antimicrobial material;
f. the carrier (B) comprises a polyamide;
g. the carrier (B) is present in an amount in the range of from 98% w/w up to and including 99.999% w/w on antimicrobial material.

2. A material according to claim 1, wherein at least 90% w/w of the particles have an underwater angle of friction equal to or lower than 30°.

3. A material according to claim 1 or claim 2, wherein the nanoparticles have an average particle size equal to or lower than 100 nm and a % degree of association lower than 50%.

4. A material according to any one of the claims 1-3, wherein the particle size of the granular material is in the range of from 1.5 mm up to and including 5.0 mm, the aspect ratio R is in the range of from 1.0 up to and including 6.0, the particles have an underwater angle of friction in the range of from 2 up to and including 28°, the amount of the antimicrobial agent (A) is in the range of from 0.001% w/w up to and including 1.8% w/w on antimicrobial material, and the amount of the carrier (B) is in the range of from 98.2% w/w up to and including 99.999% w/w on antimicrobial material.

5. A material according to any one of the claims 1-4, wherein the polyamide is selected from the group consisting of nylon-6, nylon-6,6, nylon-4,6 and mixtures thereof.

6. A method for the preparation of a granular antimicrobial material according to any one of the claims 1-5, comprising the following steps:

   a. dry-mixing a polyamide with a chemical compound comprising silver as cation, thereby forming a premix;
   b. blending and heating the premix for a time and at a temperature which are:

      i) suitable to flow out the polyamide without decomposing it;
      ii) suitable to decompose the chemical compound;

   c. extruding, cooling and cutting;
   d. collecting the granules with particle size smaller than 8 mm and greater than 1 mm.

7. An article comprising a granular antimicrobial material according to any one of the claims 1-5 and an enveloping body containing the granular material.

8. An article according to claim 7, wherein the enveloping body is a container.

9. An article according to claim 7, wherein the enveloping body is a porous water-permeable enveloping body, which porous water-permeable enveloping body has pores with a diameter such as to prohibit the granular antimicrobial material to spill out from the porous water-permeable enveloping body.

10. A device consisting of an assembly of parts, comprising one part containing an article according to claim 9 or a granular antimicrobial material according to any one of claims 1-5.

11. Use of:

   a) a granular antimicrobial material according to either any one of the claims 1-5; or
   b) an article according to any one of the claims 7-9; or
   c) a device according to claim 10,

   for water treatment and in particular for treatment of drinking water.

12. Use of a granular antimicrobial material according to any one of the claims 1-5, for antimicrobial applications, wherein the granular antimicrobial material is used in an amount and in a format that allows the granular antimicrobial material to exhibit its antimicrobial activity.

### Patentansprüche

1. Antimikrobielles Material, bestehend aus Partikeln, die ein antimikrobielles Mittel (A) und einen Träger (B) umfassen, **dadurch gekennzeichnet, dass**:

a. das antimikrobielle Material ein granulatförmiges Material ist;
b. wenigstens 90 % (Gew./Gew.) der Partikel eine Partikelgröße im Bereich von 1,0 mm bis einschließlich 8,0 mm, gemessen gemäß dem Verfahren DIN 66165-1/-2, aufweisen;
c. wenigstens 90 % (Gew./Gew.) der Partikel ein Aspektverhältnis R von gleich oder kleiner als 8 aufweisen;
d. das antimikrobielle Mittel (A) elementare Silber-Nanopartikel umfasst, welche elementaren Silber-Nanopartikel in den Träger (B) gemischt sind;
e. das antimikrobielle Mittel (A) in einer Menge im Bereich von 0,001 % (Gew./Gew.) bis einschließlich 2 % (Gew./Gew.) des antimikrobiellen Materials vorhanden ist;
f. der Träger (B) ein Polyamid umfasst;
g. der Träger (B) in einer Menge im Bereich von 98 % (Gew./Gew.) bis einschließlich 99,999 % (Gew./Gew.) des antimikrobiellen Materials vorhanden ist.

**2.** Material gemäß Anspruch 1, wobei wenigstens 90 % (Gew./Gew.) der Partikel einen Unterwasser-Reibungswinkel von gleich oder kleiner als 30° aufweisen.

**3.** Material gemäß Anspruch 1 oder Anspruch 2, wobei die Nanopartikel eine mittlere Partikelgröße von gleich oder kleiner als 100 nm und einen prozentuellen Assoziationsgrad von kleiner als 50 % aufweisen.

**4.** Material gemäß einem der Ansprüche 1-3, wobei die Partikelgröße des granulatförmigen Materials im Bereich von 1,5 mm bis einschließlich 5,0 mm liegt, das Aspektverhältnis R im Bereich von 1,0 bis einschließlich 6,0 liegt, die Partikel einen Unterwasser-Reibungswinkel im Bereich von 2 bis einschließlich 28° aufweisen, die Menge des antimikrobiellen Mittels (A) im Bereich von 0,001 % (Gew./Gew.) bis einschließlich 1,8 % (Gew./Gew.) des antimikrobiellen Materials liegt und die Menge des Trägers (B) im Bereich von 98,2 % (Gew./Gew.) bis einschließlich 99,999 % (Gew./Gew.) des antimikrobiellen Materials liegt.

**5.** Material gemäß einem der Ansprüche 1-4, wobei das Polyamid ausgewählt ist aus der Gruppe bestehend aus Nylon-6, Nylon-6,6, Nylon-4,6 und Gemischen davon.

**6.** Verfahren zum Herstellen eines granulatförmigen antimikrobiellen Materials gemäß einem der Ansprüche 1-5, umfassend folgende Schritte:

a. Trockenmischen eines Polyamids mit einer chemischen Verbindung, die Silber als Kation umfasst, um so eine Vormischung zu bilden;
b. Mischen und Erhitzen der Vormischung über eine Zeit und mit einer Temperatur, die folgendes sind:

i) geeignet, um das Polyamid auszufließen ohne es zu zersetzen;
ii) geeignet, um die chemische Verbindung zu zersetzen;

c. Extrudieren, Abkühlen und Schneiden;
d. Sammeln der Granulatkörner mit Partikelgrößen kleiner als 8 mm und größer als 1 mm.

**7.** Gegenstand, umfassend ein granulatförmiges antimikrobielles Material gemäß einem der Ansprüche 1-5 und einen umhüllenden Körper, der das granulatförmige Material enthält.

**8.** Gegenstand gemäß Anspruch 7, wobei der umhüllende Körper ein Behälter ist.

**9.** Gegenstand gemäß Anspruch 7, wobei der umhüllende Körper ein poröser wasserdurchlässiger umhüllender Körper ist, welcher poröse wasserdurchlässige umhüllende Körper Poren mit einem Durchmesser, der das Austreten des granulatförmnigen antimikrobiellen Materials aus dem porösen wasserdurchlässigen umhüllenden Körper verhindert, aufweist.

**10.** Vorrichtung, bestehend aus einem Aufbau von Teilen, umfassend einen Teil, der einen Gegenstand gemäß Anspruch 9 oder ein granulatförmiges antimikrobielles Material gemäß einem der Ansprüche 1-5 enthält.

**11.** Verwendung von:

a) einem granulatförmigen antimikrobiellen Material gemäß einem der Ansprüche 1-5; oder
b) einem Gegenstand gemäß einem der Ansprüche 7-9; oder

c) einer Vorrichtung gemäß Anspruch 10,

zur Wasserbehandlung und insbesondere zur Behandlung von Trinkwasser.

**12.** Verwendung eines granulatförmigen antimikrobiellen Materials gemäß einem der Ansprüche 1-5 für antimikrobielle Anwendungen, wobei das granulatförmige antimikrobielle Material in einer Menge und in einem Format verwendet wird, die/das dem granulatförmigen antimikrobiellen Material erlaubt, seine antimikrobielle Wirkung zu zeigen.

## Revendications

**1.** Matériau antimicrobien constitué de particules comprenant un agent antimicrobien (A) et un support (B), **caractérisé en ce que** :

a. le matériau antimicrobien est un matériau granulaire ;
b. au moins 90% en poids des particules ont une granulométrie dans la fourchette de 1,0 mm jusqu'à et y compris 8,0 mm, mesurée selon la méthode DIN 66165-1/-2 ;
c. au moins 90% en poids des particules ont un rapport d'aspect R inférieur ou égal à 8 ;
d. l'agent antimicrobien (A) comprend des nanoparticules d'argent élémentaire, lesquelles nanoparticules d'argent élémentaire sont mélangées dans le support (B) ;
e. l'agent antimicrobien (A) est présent dans une quantité dans la fourchette de 0,001% en poids jusqu'à et y compris 2% en poids par rapport au matériau antimicrobien ;
f. le support (B) comprend un polyamide ;
g. le support (B) est présent dans une quantité dans la fourchette de 98% en poids jusqu'à et y compris 99,999% en poids par rapport au matériau antimicrobien.

**2.** Matériau selon la revendication 1, dans lequel au moins 90% en poids des particules ont un angle de frottement sous l'eau inférieur ou égal à 30°.

**3.** Matériau selon la revendication 1 ou la revendication 2, dans lequel les nanoparticules ont une granulométrie moyenne inférieure ou égale à 100 nm et un % de degré d'association inférieur à 50%.

**4.** Matériau selon l'une quelconque des revendications 1-3, dans lequel la granulométrie du matériau granulaire est dans la fourchette de 1,5 mm jusqu'à et y compris 5,0 mm, le rapport d'aspect R est dans la fourchette de 1,0 jusqu'à et y compris 6,0, les particules ont un angle de frottement sous l'eau dans la fourchette de 2 jusqu'à et y compris 28°, la quantité de l'agent antimicrobien (A) est dans la fourchette de 0,001% en poids jusqu'à et y compris 1,8% en poids par rapport au matériau antimicrobien, et la quantité du support (B) est dans la fourchette de 98,2% en poids jusqu'à et y compris 99,999% en poids par rapport au matériau antimicrobien.

**5.** Matériau selon l'une quelconque des revendications 1-4, dans lequel le polyamide est choisi dans le groupe constitué par le nylon-6, le nylon-6,6, le nylon-4,6 et leurs mélanges.

**6.** Procédé de préparation d'un matériau granulaire antimicrobien selon l'une quelconque des revendications 1-5, comprenant les étapes suivantes :

a. le mélangeage à sec d'un polyamide avec un composé chimique comprenant de l'argent comme cation, afin de former un prémélange ;
b. le mélangeage et le chauffage du prémélange pendant une durée et à une température qui sont :

i) appropriées pour faire s'écouler le polyamide sans le décomposer ;
ii) appropriées pour décomposer le composé chimique ;

c. l'extrusion, le refroidissement et le découpage ;
d. le recueil des granulés ayant une granulométrie inférieure à 8 mm et supérieure à 1 mm.

**7.** Article comprenant un matériau granulaire antimicrobien selon l'une quelconque des revendications 1-5 et un corps enveloppant contenant le matériau granulaire.

**8.** Article selon la revendication 7, dans lequel le corps enveloppant est un récipient.

**9.** Article selon la revendication 7, dans lequel le corps enveloppant est un corps enveloppant poreux perméable à l'eau, lequel corps enveloppant poreux perméable à l'eau possède des pores ayant un diamètre tel qu'il empêche le matériau granulaire antimicrobien de s'échapper du corps enveloppant poreux perméable à l'eau.

**10.** Dispositif constitué d'un assemblage de composants, comprenant un composant contenant un article selon la revendication 9 ou un matériau granulaire antimicrobien selon l'une quelconque des revendications 1-5.

**11.** Utilisation :

a) d'un matériau granulaire antimicrobien selon l'une quelconque des revendications 1-5 ; ou
b) d'un article selon l'une quelconque des revendications 7-9 ; ou
c) d'un dispositif selon la revendication 10,

pour le traitement de l'eau et en particulier pour le traitement de l'eau potable.

**12.** Utilisation d'un matériau granulaire antimicrobien selon l'une quelconque des revendications 1-5, pour des applications antimicrobiennes, dans laquelle le matériau granulaire antimicrobien est utilisé dans une quantité et dans un format qui permettent au matériau granulaire antimicrobien de manifester son activité antimicrobienne.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5011602 A
- WO 2009073994 A


### Non-patent literature cited in the description


- Silver in Drinking Water. Guidelines for drinking-water quality. 1996, vol. 2
- **C. K. WENTWORTH.** A scale of grade and class terms for clastic sediments. *J. Geology,* 1922, vol. 30, 377-392